# EUROPEAN PATENT APPLICATION

(11) **EP 1 728 873 A1**
(43) Date of publication of application: **06.12.2006**
(21) Application number: 05011599.7
(22) Date of filing: 30.05.2005
(51) Int. Cl.: C12Q 1/68

(54) **Use of an insulinoma cell for identifying pancreatic beta-cell mitogens**

(71) Applicant: DeveloGen Aktiengesellschaft, 37079 Göttingen (DE)
(72) Inventor: The designation of the inventor has not yet been filed
(74) Representative: Weiss, Wolfgang

(57) **Abstract**

The present invention relates to a method of identifying and/or characterizing pancreatic beta-cell mitogens by using cells expressing a pancreatic gene or a gene whose function is controlled by a pancreatic gene, particularly the Pax4 gene, and which are transfected with a reporter gene.

## Description

The present invention relates to a method of identifying and/or characterizing pancreatic beta-cell mitogens by using cells expressing a pancreatic gene or a gene whose function is controlled by a pancreatic gene, particularly the Pax4 gene, and which are transfected with a reporter gene.

Pancreatic beta-cells secrete insulin in response to blood glucose levels. Insulin amongst other hormones plays a key role in the regulation of the fuel metabolism. Insulin leads to the storage of glycogen and triglycerides and to the synthesis of proteins. The entry of glucose into muscles and adipose cells is stimulated by insulin. In patients who suffer from diabetes mellitus type I or LADA (Latent Autoimmue Diabetes in Adults, Pozzilli & Di Mario, 2001, Diabetes Care. 8:1460-67) beta-cells are being destroyed due to autoimmune attack. The amount of insulin produced by the remaining pancreatic islet cells is too low, resulting in elevated blood glucose levels (hyperglycemia). In diabetes type mellitus II liver, fat and muscle cells loose their ability to respond to normal blood insulin levels (insulin resistance). High blood glucose levels (and also high blood lipid levels) in turn lead to an impairment of beta-cell function and to an increase in beta-cell apoptosis. It is interesting to note that the rate of beta-cell neogenesis does not appear to change in type II diabetics (Butler et al., 2003 Diabetes 52: 102-110,), thus causing a reduction in total beta-cell mass over time. Improving metabolic parameters such as blood sugar and blood lipid levels (e.g. trough dietary changes, exercise, medication or combinations thereof) often leads to a transient restoration of beta-cell function. However, the application of exogenous insulin eventually becomes necessary in type II diabetics.

In type I diabetics, where beta-cells are being destroyed by autoimmune attack, treatments have been devised which modulate the immune system and may be able to stop or strongly reduce islet destruction (Raz et al., 2001, Lancet 358: 1749-1753; Chatenoud et al., 2003, Nat Rev Immunol. 3: 123-132; Homann et al., Immunity. 2002, 3:403-15). The use of immunomodulatory agents in combination with therapeutics that trigger beta cell growth is expected to efficiently restore beta cell mass and thereby further improve glycemic control in T1 D or LADA patients.

Diabetes is a very disabling disease, because today's common anti-diabetic drugs do not control blood sugar levels well enough to completely prevent the occurrence of high and low blood sugar levels. Out of range blood sugar levels are toxic and cause long-term complications like for example renopathy, nephropathy, neuropathy and peripheral vascular disease. Therapy approaches can also lead to episodes of dangerously low blood glucose levels which may lead to coma and death. There is also a host of related conditions, such as obesity, hypertension, heart disease and hyperlipidemia, for which persons with diabetes are substantially at risk.

Apart from the impaired quality of life for the patients, the treatment of diabetes and its long term complications presents an enormous financial burden to our healthcare systems with rising tendency. Thus, for the treatment of diabetes mellitus type I and LADA, but also for late stages of diabetes mellitus type II there is a strong need in the art to identify factors that induce regeneration of pancreatic insulin producing beta-cells. These factors could restore normal function of the endocrine pancreas once its function is impaired or even could prevent the development or progression of diabetes mellitus type I, late stage diabetes mellitus type II or LADA. Even modest increases in beta cell mass will lead to a reduction both in long term diabetic complications and hypoglycemic episodes (Steffes et al., 2003, Diabetes Care 26: 832-836).

Thus, there is a need to identify and/or characterize mitogens of pancreatic beta-cells.

The identification of novel factors that are able to induce selectively the proliferation of beta cells is a difficult task. Mitogens are often not selective and are either able to induce proliferation in a range of different cell types or have other pleiotrophic effects on cells which in case of a systemic treatment could be harmful for patients. In the art primary beta cells have been used to identify and characterize beta-cell mitogens. However, the numbers of beta-cells per animal (rat or mouse) is comparatively small and the isolation of islets from animals as well as the cultivation of primary beta-cells is technically demanding making them unsuitable for screening approaches e. g. in combination with chemical libraries. Alternatively, pancreatic insulin producing cell lines have been used by researchers for searching beta-cell mitogens as exemplified in the publication by Huotari and co-workers (Huotari et al., Endocrinology, 1998, 139: 1494-1499). Though this approach has yielded interesting results, it is hampered by the fact that in contrast to the vast majority of primary beta-cells immortalized cells are in cycle, probably due to defects in the regulation of their cell cycle or other intracellular signalling pathways. It is therefore questionable, if results generated with such cell lines reflects the situation in primary beta cells.

Previous findings show that Pax4 expression promotes beta cell differentiation during development (Sosa-Pineda et al. (1997), Nature 386:399-402) or stem cell differentiation (Blyszczuk et al. (2003) PNAS 100: 998-1003, patent application WO02/086107), as well as proliferation and survival of mature beta-cells (Brun et al. (2004), J. Cell Bio. 167:1123-1135).

Brun et al. describe the β-cell proliferation and survival in rat and human islets of Langerhans through the influence of the β-cell transcription factor Pax4.

US 2003/0138948 discloses the determination of phenotypic or metabolic changes in a cell population obtained by differentiating primate pluripotent stem cells after stimulation with a test compound.

In FEBS Lett. 480 (2000): 101-105 Ueda describes the characterization of activin A as a β-cell mitogen capable of stimulating Pax4 expression. Either the Pax4 transcript or expression of a reporter gene is determined. The reporter gene construct comprises activin response elements or the human insulin promoter as expression control sequences operatively linked to a luciferase gene and is expressed in murine NIT1 or rat INS-1 E cells.

Thus, the technical problem underlying the present invention is to provide a new and efficient method for identifying and/or characterizing pancreatic beta-cell mitogens which may be used as drugs or drug candidates for the prevention or treatment of pancreatic disorders including diabetes. The solution to said technical problems is achieved by providing the embodiments characterized in the claims.

Accordingly, the present invention relates to a method of identifying and/or characterizing beta-cell mitogens by using a cell transfected with a reporter gene construct comprising an expression control sequence, e.g. a pancreatic expression control sequence such as the Pax4 expression control sequence operatively linked to a reporter gene.

In particular, the invention relates to a method of identifying and/or characterizing a pancreatic beta-cell mitogen comprising the steps:
(i) providing a cell which is transfected with a reporter gene construct comprising a reporter gene which is operatively linked to an expression control sequence of a pancreatic gene or a gene whose function is controlled by a pancreatic gene, preferably the Pax4 gene,
(ii) contacting said cell with a compound and
(iii) determining reporter gene expression in said cell as a response to the presence of the compound.

The cell capable of regulating the expression level of a pancreatic gene or a gene whose function is controlled by a pancreatic gene, particularly the Pax4 gene, is preferably of pancreatic origin, derived from a beta cell or a precursor thereof, an insulinoma or insulinoma derived cell. The cell is preferably of mammalian origin such as a rat cell, e.g. INS-1 (Asfari et al. (1992), Endocrinology 130:167-178), mouse cell, e.g. a NIT-1 cell (Hamaguchi et al. (1991), Diabetes 40: 842-849), or a human cell, in which the expression of the endogenous Pax4 is inducible, e.g. by activins or betacellulin (Ueda (2000), FEBS Lett. 480:101-105), and which have been transfected with a suitable reporter construct. Especially preferred are transgenic cell lines containing said reporter construct which exhibits increased Pax4 reporter gene activity after treatment with activators like activin, betacellulin or kinase inhibitors, such as INS-1-cl.-1.5 or INS-1-cl.-9.

The reporter gene construct comprises an expression control sequence, and optionally part of the gene locus, of a pancreatic gene or a gene whose function is controlled by a pancreatic gene, preferably the Pax4 promoter and the Pax4 gene locus. Further non-limiting examples of pancreatic genes are Pdx1, Pax-4, Pax-6, neurogenin 3 (ngn3), Nkx6.1, Nkx6.2, Nkx2.2, HB9, BETA2/NeuroD, Isl1, HNF1-alpha, HNF1-beta, HNF3, HNF4 alpha, Hes1 and H1xb9 or IRS2, c-myc, a cyclin, a CDK inhibitory protein, Menin1 and CDK4 of mammalian or human origin.

The reporter gene may be any gene expressing a reporter gene product which gives a phenotypically detectable signal, e.g. a signal which can be detected by optical or enzymatic methods. Preferred examples of reporter genes are the firefly luciferase gene, the chloramphenicol transferase gene (CAT) or beta galactosidase gene (Current Protocols In Molecular Biology, Ausubel, I., Frederick, M. (1999); John Wiley & Sons, Inc.; Introduction of DNA into Mammalian Cells: Overview of Genetic Reporter Systems; page 9.6.3). The reporter gene is heterologous to the expression control sequence.

Preferably, the inventive method is performed *in vitro,* e.g. in a cell culture system. The method may also be performed *in vivo* in a non-human transgenic animal.

The reporter gene construct may be inserted into an appropriate vector, i.e. a vector which allows propagation and expression in an insulin producing cell. Methods which are well known to those skilled in the art may be used to construct suitable vectors containing sequences encoding the proteins and the appropriate transcriptional and translational control elements. Such techniques are described in Sambrook, J. et al. (1989) Molecular Cloning, A Laboratory Manual, Cold Spring Harbor Press, Plainview, N. Y. And Ausubel, F. M. et al. (1989) Current Protocols in Molecular Biology, John Wiley & Sons, New York, N. Y.

Appropriate mammalian expression vectors for efficient expression, selection, and analysis of recombinant proteins are well known in the art (Sambrook, J. et al. (1989) Molecular Cloning, A Laboratory Manual, Cold Spring Harbor Press, Plainview, N. Y.), such as vector systems based on viruses such as retroviruses or adenoviruses. Another type of vector that is suitable for transfection is known in the art as plasmid expression or cloning vectors, e. g. Bluescript vectors (Stratagene) or plasmid pCMV-SPORT (Invitrogen), is commercially available from a number of different suppliers.

The reporter gene construct can be either transiently or stably inserted into the cell by any suitable method. Transfection methods are well known in the art (see, for example, Ausubel et al., 1991, Current Protocols in Molecular Biology, Wiley and Sons, New York). Preferably, the cell is stably transfected with the reporter gene construct.

The transfected cells according to the present invention are contacted with a test compound under conditions wherein the effect of the test compound on the reporter gene product expression can be determined. Preferably at least 10⁴ cells, more preferably at least 2 x 10⁴ cells are used for each test. The test compound may be dissolved in a buffer, medium or solvent which is physiologically acceptable for the cells and then incubated with the cells for a suitable time period, e.g. of about 1 hour to about 80 hours, preferably from about 3 hours to about 60 hours, most preferably about 6 to about 48 hours.

After the contacting step, the reporter gene expression is determined. The determination comprises a qualitative and/or quantitative detection of a gene product which is formed upon expression of the reporter gene. If an increased reporter gene expression versus a control, e.g. reporter gene expression in the absence of a test compound, is found, the test compound can act as a pancreatic beta-cell mitogen. The reporter gene expression may be determined by any suitable optical or enzymatic method well known in the art.

The present invention is suitable for automatised cell-based (high through-put and ultra high through-put) screening assays which are well known in the art. With the present method, a plurality of compounds can be screened in parallel within a very short time.

Surprisingly it was found that the method disclosed in the present invention is easy, accurate and quick.

Insulinoma derived cells are valuable cellular systems for identifying and/or characterizing pancreatic beta-cell mitogens. Molecules capable of inducing the expression of a pancreatic gene or a gene whose function is controlled by a pancreatic gene, such as Pax4 in pancreatic beta-cells or precursors thereof are putative beta cell mitogens. Cells, e. g. insulinoma cells that are transfected with an inducible Pax4 gene are among the best cellular systems to identify and/or characterize beta cell mitogens, in particular when the Pax4 expression can be induced by physiological stimuli, such as with activins, beta-cellulin, prolactin, glucose or GLP-1, because these cells are expected to comprise Pax4 regulative signalling pathways alike real beta-cells.

Thus, molecules activating pancreatic gene expression or the expression of a gene whose function is controlled by a pancreatic gene such as Pax4 expression in said cells, e. g. insulinoma cells, are also expected to be effective in primary beta-cells and *in vivo.* The invention described herein facilitates the identification and/or characterization of novel pancreatic beta-cell mitogens, e. g. it can be used to screen large chemical or compound libraries for beta cell mitogens, particularly Pax4 agonists.

Unless defined otherwise, all technical and scientific terms used in this specification are considered to have the same meaning as commonly understood by persons of ordinary skill in the art to which the present invention pertains.

The term "beta-cell mitogen" as used herein refers to a compound that is capable of modulating the differentiation or regeneration of cells into funtional pancreatic beta-cells, particularly insulin-producing cells. Moreover, the term "beta-cell mitogen" encompasses molecules that trigger mitosis in beta-cells or precursors thereof. The term "beta-cell mitogen" as used herein further describes all molecules that are able to increase the beta-cell mass in vitro or in vivo.

The term "beta cell regeneration" refers to an increase in beta cell mass compared to the untreated or control treated situation or a delay in beta cell loss. It may be brought about by an increase in beta cell replication, reduced beta cell death, increased beta cell formation from non-beta cells or combinations thereof.

The term "expression control sequence" refers to a sequence which can affect the ability of transcriptional regulatory proteins (transcription factors) and RNA polymerases to find access to specific genes and to activate transcription from them. Examples are promoter and/or enhancer sequences.

A "compound" refers to any natural or synthetic molecule with the capability of altering or mimicking the physiological function of a beta-cell mitogen. Compounds encompass numerous chemical classes, though typically they are organic molecules, preferably small molecules. Compounds are also found among biomolecules, including nucleic acids such as antisense molecules, RNAi reagents, e.g. double-stranded RNA molecules, single-stranded RNA molecules or DNA molecules encoding the latter, cDNA expression constructs, genomic expression constructs, peptides or proteins. Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc, to produce structural analogues. Of particular interest are compounds selected from nucleic acids such as RNA inhibiting (RNAi) oligonucleotides.

The term "pancreatic gene" refers to a gene that is involved and required for pancreas development, more preferably beta-cell differentiation. Examples of pancreatic genes are Pdx1, Pax-4, Pax-6, neurogenin 3 (ngn3), Nkx6.1, Nkx6.2, Nkx2.2, HB9, BETA2/NeuroD, Isl1, HNF1-alpha, HNF1-beta, HNF3, Hes1, H1xb9 and HNF4 alpha. A gene whose function is controlled by a pancreatic gene, for example, refers to a transcription target such as IRS2, C-myc, a cyclin, a CDK inhibitory protein, Menin1 or CDK4.

A "reporter gene" refers to a gene sequence whose phenotypic expression, that is expressing a reporter gene product (reporter), can be monitored. It may be used to study expression control sequence activity in different tissues or developmental stages. The reporter gene is operatively linked to an expression control sequence such as a promoter region of particular interest. The resulting construct is suitable for transfection into a cell or organism.

Compounds which are identified and/or characterized to have beta-cell mitogenic activity within the present invention are suitable for the preparation of a medicament for treating a pancreatic disorder such as diabetes mellitus. Thus, in a preferred embodiment, the inventive method further comprises the production of a pharmaceutical preparation.

It was shown that RNAi oligonucleotides can activate the reporter gene construct as disclosed in the present invention. Thus, in a preferred embodiment, the inventive method is also suitable for a genome spanning RNAi oligonucleotide screening for the identification of sequences which directly or indirectly regulate the expression of a pancreatic gene or a gene whose function is controlled by a pancreatic gene such as a negative regulatory sequence and/or an inhibitory gene.

A further aspect of the present invention relates to an insulinoma cell which is transiently or stably transfected with a reporter gene which is operatively linked to an expression control sequence and, optionally, part of the gene locus of a pancreatic gene or a gene whose function is controlled by a pancreatic gene. Preferably, the reporter gene comprises the Pax4 promoter and the Pax4 gene locus, as well as a reporter gene sequence, particularly of human origin.

An even further aspect of the present invention relates to a test system for the identification and characterization of a beta-cell mitogen comprising an insulinoma derived cell which is stably transfected with a reporter gene which is operatively linked to an expression control sequence of a pancreatic gene or a gene whose function is controlled by a pancreatic gene, preferably the Pax4 gene, and at least one positive or negative control.

In a preferred embodiment, the expression control sequence of the test system is of mammalian, preferably human origin. The reporter gene of the test system comprises a sequence coding for, amongst others, a firefly luciferase, chloramphenicol acetyltransferase or a beta galactosidase gene (Current Protocols In Molecular Biology, Ausubel, I., Frederick, M. (1999); John Wiley & Sons, Inc.; Introduction of DNA into Mammalian Cells: Overview of Genetic Reporter Systems; page 9.6.3).

In a further preferred embodiment, the control compounds are selected from, amongst others, activin A, B, AB, C or D, TGF beta, HGF, IGF, prolactin, GLP-1 or derivatives thereof, EGF, betacellulin, glucose or a small molecule kinase inhibitor such as inhibitor I, II or III, or combinations thereof.

It should be noted that all preferred embodiments discussed for one or several aspects of the invention also relate to all other aspects.

The following figures and examples illustrate the invention and are non-limiting embodiments of the invention as claimed below. Numerous additional aspects and advantages of the invention will become apparent to those skilled in the art upon consideration of the following description of the figures which describes presently preferred embodiments thereof.

### Figure Legends

**Figure 1** shows the Pax4 RNA expression level in insulinoma INS-1E cells without treatment (Co) or after treatment with activin A, TGF-beta, activin B, activin AB, BMP-4 and BMP-7 in concentrations as indicated. Pax4 expression levels were quantitatively determined by real time RT-PCR and are indicated in relative amounts.
**Figure 2A** shows the Pax reporter gene construct comprising the Pax4 promotor sequence from -6500 to +1 which was ligated upstream of a firefly luciferase (*LUC*) reporter gene, and 7,8 kb of the genomic Pax4 gene locus. The 17,7 kb cDNA construct was cloned into pBlueskript KS. The numbers 1 to 10 indicate human Pax4 exons. Figure 2B shows a schematic illustration of an INS-1E insulinoma cell carrying a Pax4-reporter gene construct.
**Figure 3** shows the induction of reporter gene (luciferase) activity in the cell INS-1-cl.-1.5 by several test compounds.
**Figure 4** shows the activity in the transgenic cell line INS-1-cl.-3.5 after treatment with activin-A. The best signal to noise ratio is achieved when 0,5x10⁵ to 1x 10⁵ cells are seeded per well (0,3 cm²) of a so-called 96 well plate.
**Figure 5** shows the induction of reporter gene activity in the cell line INS-1-cl.-3.5 by treatment with activin-A, TGF-beta and kinase inhibitor II.

The versatility of the present invention is illustrated, but not limited, by the following examples.

### Example 1: Generation of stable cell lines

INS-1E cell lines were grown as described below. The Pax4-reporter gene construct (referred to as pKS-Pax4-LUC), a cDNA construct containing a firefly luciferase reporter (*LUC*) gene under the transcriptional control of human Pax4 promoter sequences and the nearly complete human Pax4 gene locus were cloned into the plasmid pBluescript ks.

Adherent INS-1E cells were transfected using Lipofectamine 2000 (Invitrogen) according to the protocol provided by the supplier. The transfected cells were treated with media containing 400 µg/ml G418 Sulfate (e.g. Geneticin from Gibco; Cat. No. 11811-098), a selection agent for eukaryotic cells, 24 to 48 hours after the transfection. To establish independent stable cell lines, cell colonies which occurred 2 to 6 weeks after the transfection were individually transferred to new culture dishes and then propagated like untransfected INS-1 E cells, but in the presence of G418 Sulfate.

### Example 2: Activin B increases Pax4 transcription

The response of the Pax4 gene to mitogens was investigated in the rat insulinoma cell line INS-1E. INS-1E cells are known to express Pax4 and to upregulate Pax4 levels in response to the treatment with activin-A and betacellulin (Ueda (2000), supra, Li et al. (2004) supra, Brun et al. (2004), supra). In the search for novel beta-cell mitogens the inventors treated INS-1 E cells with proteins related to activin A or betacellulin. Activin B an activin AB were found to be almost equally potent in stimulating Pax4 transcription as activin A. Other TGF-beta family members, such as BMP 4 and 7 which are known to recognize the activin-receptor type II subunit of the heterodimeric activin receptor hardly induced Pax4 gene transcription. Maximal Pax4 induction was observed with 1 nM activin B that induced about a 7.5-fold increase in Pax4 levels. The Pax4 RNA expression level was normalized to this of 18S RNA. The level of the unrelated gene RNA polymerase II largest subunit (RPB1) was unaffected by activin B treatment.

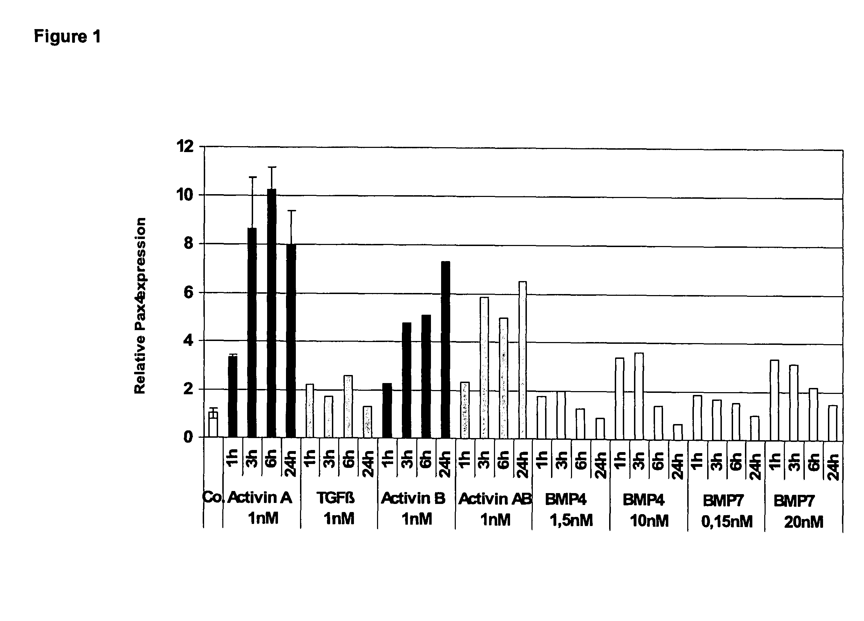

Fig.1 illustrates a representative experiment in which the relative Pax4 levels were quantified using quantitative real time RT-PCR. As shown in Fig. 1, activin B and activin AB induce Pax4 gene transcription in INS-1E cells. Quantitative real-time RT-PCR was done with RNA isolated from INS-1E cells cultured under conditions as described below. Low levels of Pax4 are expressed in INS-1E cells. Data are presented as relative levels to the basal Pax4 expression level in untreated INS-1E cells (Co.). The values for untreated INS-1E (Co.) and activin-B are averages of three experiments enabling the determination of standard deviations; the other values are averages of two experiments.

### Quantitative RT-PCR

Total RNA from 8x10⁴ cells growing on 4 cm² surface area of a tissue culture dish was extracted using Qiagen RNAeasy kit according to the instructions of the manufacturer (Qiagen) and 2 µg was converted into cDNA. Primers for pax4, 18S RNA, and rat RNA polymerase II largest subunit (RPB1) were designed using the Primer Express 1.5 Software from Applied Biosystems and sequences can be obtained upon request. Quantitative real-time PCR was performed using Applied Biosystems SDS 7000 detection system. Amplifications from 2 independent experiments were performed in duplicate for each transcript and mean values were normalized to the mean value of the reference RNA 18S RNA.

### Cell culture

INS-1E cells were cultured as described (Merglen, (2004) Endocrinology;145: 667-678). Cells were seeded at a density of 2x10⁴ cells per cm² 6 to 8 days before the treatment with chemicals. During the growth period the medium was changed once. The cells were incubated for different periods of time with chemicals under serum-free conditions. The cells were harvested in Qiagen RNA easy cell lysate buffer and immediately transferred to dry ice. The samples were stored at -20 degree until RNA isolation was carried out.

### Example 3: Identification and/or characterization of beta-cell mitogens

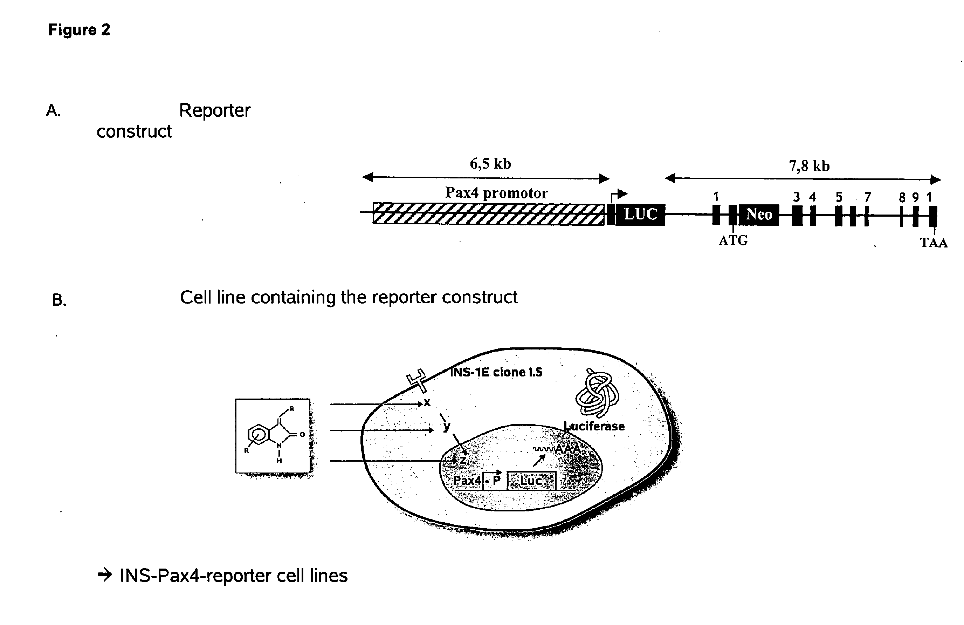

For the identification and/or characterization of beta-cell mitogens a Pax4 reporter gene assay has been established. The rat insulinoma cell line INS-1 E was stably transfected with a DNA construct containing the luciferase reporter gene under the transcriptional control of the human Pax4 promoter and the complete Pax4 gene locus (Figure 2).

Luciferase activity that can be quantified using appropriate imaging systems reflects the activation status of the human Pax4 promoter. Two types of cell lines reacting either to activins or small molecule kinase inhibitors have been identified so far.

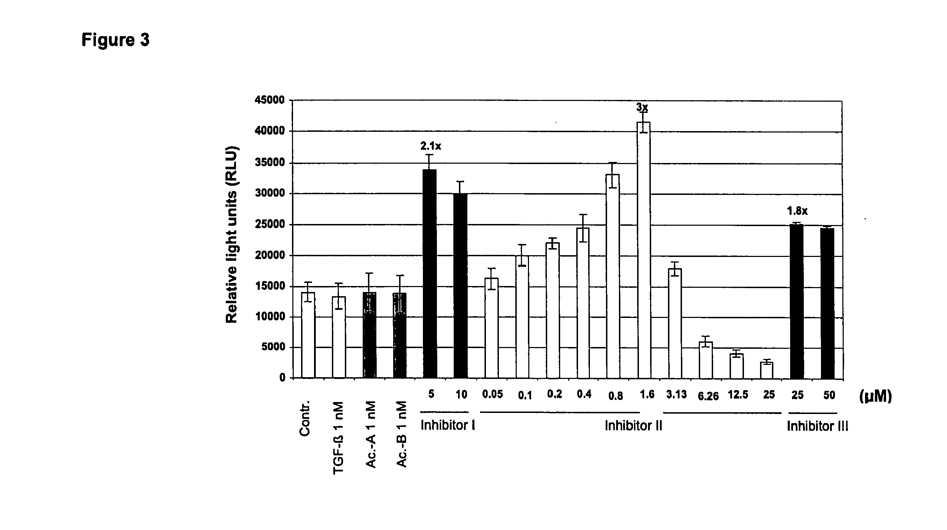

INS-1-cl-1.5 cells show Pax4 reporter gene activity upon treatment with small molecule kinase inhibitors as exemplified in Figure 3. Maximal Pax4 reporter gene activity observed was about 3-fold above the level of untreated cells (Contr.) after 48 hour treatment with inhibitor II. Similar levels of activation were observed after 24 and 62 hours of incubation with inhibitor II (data not shown). The assay signal dynamic range is between 2 and 3. This clone, however, does not react to Activin stimulation indicating the existence of at least two independent signalling pathways regulating Pax4 transcription.

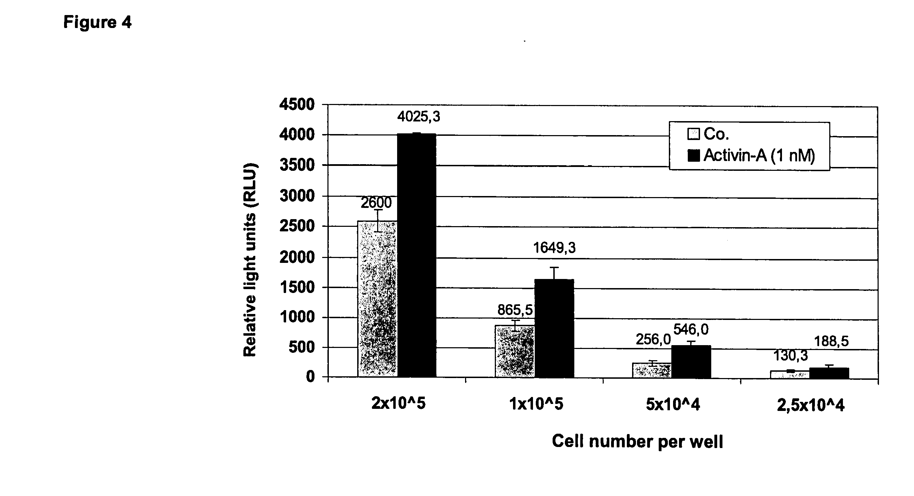

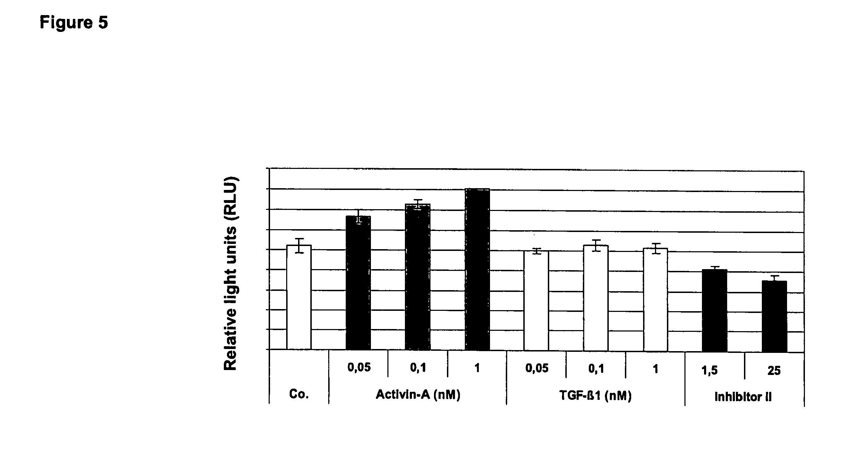

The second type of clones identified react to Activin treatment but not to incubation with the kinase inhibitors II (Figure 5), II and III (data not shown). Two independent clones named INS-1-cl.-3.5 or INS-1-cl.-9 showed this pattern of Pax4 regulation. The reason for the different regulation of the human Pax4 promoter observed in the different cell lines is unknown. Here, representative data generated with the cell line INS-1-cl.-3.5 are presented. Most importantly, the treatment of the reporter cell line with the Activin related protein TGF-beta 1 does not effect Pax4 transcription (Figure 4). The assay signal dynamic range is somewhere between 0.5 and 1 (Figure 4).

## Claims

1. A method of identifying and/or characterizing a pancreatic beta-cell mitogen comprising the steps:
(i) providing a cell which is transfected with a reporter gene construct comprising a reporter gene which is operatively linked to an expression control sequence of a pancreatic gene or a gene whose function is controlled by a pancreatic gene, preferably the Pax4 gene,
(ii) contacting said cell with a compound and
(iii) determining reporter gene expression in said cell as a response to the presence of the compound.

2. The method of claim 1, wherein said cell is capable of regulating the expression level of a pancreatic gene or a gene whose function is controlled by a pancreatic gene and is preferably of pancreatic origin, derived from a beta cell or a precursor cell thereof.

3. The method of claims 1 or 2, wherein said cell is an insulinoma cell, preferably of mammalian origin.

4. The method of any one of claims 1 to 3, wherein said cell is a cell which is responsive to treatment with kinase inhibitors and/or to treatment with activin A, B, AB, C, D, TGF-beta, HGF, IGF, prolactin, GLP-1 or derivatives thereof, EGF, betacellulin, glucose.

5. The method of any one of claims 1 to 4, wherein said expression control sequence is of mammalian, preferably human, origin.

6. The method of any one of claims 1 to 5, wherein said reporter gene encodes a gene product which can be determined by optical or enzymatic methods.

7. The method of any one of claims 1 to 6, wherein said reporter gene comprises a sequence coding for firefly luciferase, chloramphenicol acetyltransferase or beta galactosidase.

8. The method of any one of claims 1 to 7, wherein said insulin producing cell is stably transfected with the reporter gene construct.

9. The method of any one of the claims 1 to 8, wherein a plurality of compounds is tested in parallel.

10. The method of any one of claims 1 to 9, wherein at least one step is carried out automatically.

11. The method of any one of claims 1 to 10, further comprising preparing a pharmaceutical preparation which comprises as an active ingredient a pancreatic beta-cell mitogen identified and/or characterized according to steps (i) ― (iii) or a compound derived therefrom.

12. Insulinoma cell which is transfected with a reporter gene construct comprising a reporter gene which is operatively linked to an expression control sequence of a pancreatic gene or a gene whose function is controlled by a pancreatic gene, preferably the Pax4 gene, preferably comprising a Pax4 promoter and at least part of the Pax4 gene locus.

13. The cell of claim 12, wherein said expression control sequence is of mammalian, preferably human origin.

14. The cell of claims 12 or 13, wherein said insulinoma cell is of mammalian origin, preferably of rat, mouse or human origin.

15. The cell of any one of claims 12 to 14 which is responsive to treatment with kinase inhibitors and/or to treatment with activin A.

16. The cell of any one of claims 11 to 15, which is stably transfected.

17. The cell of any one of the claims 11 to 16, wherein said reporter gene comprises a sequence coding for firefly luciferase, chloramphenicol acetyltransferase or beta galactosidase.

18. Test system comprising an insulinoma cell which is stably transfected with a reporter gene construct comprising a reporter gene operatively linked to an expression control sequence of a pancreatic gene or a gene whose function is controlled by a pancreatic gene preferably the Pax4 gene and at least one positive or negative control compound.

19. The test system of claim 18, wherein said expression control sequence is of mammalian, preferably human, origin.

20. The test system of claim 18 or 19, wherein said reporter gene comprises a sequence coding for firefly luciferase, chloramphenicol acetyltransferase or beta galactosidase.

21. The test system of any one of claims 18 to 20, wherein said positive or negative control compounds are selected from activins A, B, AB, C, D, TGF-beta, HGF, IGF, prolactin, GLP-1 or derivatives thereof, EGF, betacellulin, glucose.small molecule kinase inhibitor, inhibitor I, II, II or a combination thereof.
